# EUROPEAN PATENT SPECIFICATION

(11) **EP 0 324 574 B1**
(45) Date of publication and mention of the grant of the patent: **18.11.1993**
(21) Application number: 89300174.3
(22) Date of filing: 10.01.1989
(51) Int. Cl.: A61K 31/365, A61K 37/02

(54) **Immunomodulating bryostatins**
Immunmodulierende Bryostatine
Bryostatines comme immunomodulants

(30) Priority: 13.01.1988 US 144912
(43) Date of publication of application: 19.07.1989
(73) Proprietor: ARIZONA BOARD OF REGENTS, Tempe Arizona 85287 (US)
(72) Inventor: Pettit, George Robert, Paradise Valley Arizona 85253 (US); Sitkovsky, Michail, V., Bethesda Maryland 20205 (US)
(74) Representative: Coxon, Philip

(56) References cited:
- EP-A- 0 109 811
- DE-A- 3 704 389
- Immunobiology, vol. 175, no. 5, Nov. 1987, pp. 420-430; H. Mohr et al.
- The J. of Immunobiology, vol. 140, no. 2, Jan. 15, 1988, pp. 433-439, The Am. Ass. of Immunologists, US; G. Trenn et al.
- The J. of Immunology, vol. 141, no. 10, Nov. 1988, pp. 3263-3269, The Am. Ass. of Immunologists, US; A.D. Hess et al.
- Arzneimittelforschung, vol. 35, no. 11, 1985, pp. 1607-1615; J.E. Kolitz et al.
- Therapiewoche, vol. 33, 1983, pp. 1291-1300; R. Mohr

## Description

The present invention relates to novel immunomodulating agents and more particularly to a novel series of protein kinase C activators which are not tumor promoters.

Recent work in cellular activation studies (See: Berridge, M. J. 1984. Inositol triphosphate and diacylglycerol as second messengers. Biochem. J. 220:345; Kikkawa, U., et al,. 1983. Protein kinase C as a possible receptor protein of tumor-promoting phorbol esters. J. Biol. Chem. 258:11442.; Nishizuka, Y. 1984. The role of protein kinase C in cell surface signal transduction and tumor promotion. Nature 308:693) and the biochemical pathways involved in the T-cell antigen receptor (TcR) mediated T-lymphocyte stimulation (See: Weiss, et al. 1984. Role of T3 surface molecules in human T-cell activation: T3-dependent activation results in an increase in cytoplasmic free calcium. Proc. Natl. Acad. Sci. USA 81:4169; Imboden, J.B., et al. 1985. Transmembrane signalling by the T-cell antigen receptor. Perturbation of the T-3 antigen receptor complex generates inositol phosphates and releases calcium ions from intracellular stores. J. Exp. Med. 161:446; Trunch, A., et al. 1985. Early steps of lymphocyte activation bypassed by synergy between calcium ionophores and phorbol ester. Nature 313:318; Utsunomiya, N., et al. 1986. Early transmembrane events in alloimmune cytotoxic T-lymphocyte activation as revealed by stopped-flow fluorometry. Proc. Natl. Acad. Sci. USA 83:1877; Berrebi, G., et al. 1987. The antigen-receptor requirement for conjugate formation and lethal hit triggering by cytotoxic T-lymphocytes can be bypassed by protein kinase C activators and Ca⁺⁺ -ionophores. Proc. Natl. Acad. Sci. USA 84:1364.) have created a new intellectual basis for the design and development of immunomodulating agents.

Of particular interest is the evaluation of immunomodulating properties of well defined agents that interfere with the Ag-receptor mediated transmembrane signalling pathway in T-lymphocyte activation. Numerous studies have shown that protein kinase C (See: Nishizuka, supra) is involved in the activation of helper (See: Imboden, et al and Trunch, et al, supra) and cytotoxic (See: Utsunomiya, et al and Berrebi, et al, supra) T-lymphocytes. The discovery of the link between inositol phospholipid metabolism and activation of protein kinase C (a major cellular receptor for phorbol esters) helps explain the effect of phorbol esters in lymphocyte activation. Phorbol esters might therefore be considered as excellent candidates for evaluation as immunomodulating agents if they were not potent tumor promoters (See: Jeffery, A. M., 1986. Computer-assisted molecular modelling of tumor promoters: Rationale for the activity of phorbol esters, teleocidin B, and aplysiatoxin. Proc. Natl Acad. Sci. USA 83:241).

Accordingly, a need exists for the identification of non-tumor promoting activators of protein kinase C which can be tested for the ability to enhance or suppress the immune response. The present invention is directed to the identification and use of a protein kinase C activator which not only is non-tumor promoting, it has been found to possess potent anti-neoplastic properties in several in vitro and in vivo systems. The specific compounds which have been discovered to meet the foregoing requirements were found in a newly discovered family of biosynthetic products contained in marine animals of the phylum Ectoprocta (familiarly name Bryozoa) and named "bryostatins". In the murine P388 lymphocyte leukemia (PS) system bryostatin 1 (NSC 339555) showed 52-96% life extension at 10-70 µg/kg injection dose levels and an ED₅₀ of 0.89 µg/ml against the P388 in vitro cell line (See: Pettit, G.R., et al. 1982. Isolation and structure of bryostatin 1. J. Amer. Chem. Soc. 104:6846). Furthermore, bryostatin 1 was found to afford 31-68% life extension at 5-40 g/kg in the M531 murine ovary sarcoma system (See: Pettit, G.R., 1984. Structure of Bryostatin 4. An important antineoplastic constituent of geographically diverse bugula neritina (Bryosoa). J. Amer. Chem. Soc. 106:6768). Similarly, bryostatins 1 and 2 have been found to be antagonists of tumor promoters.

The present invention is based on our discovery of the stimulatory effects of bryostatins 1 and 2 on the development and effector functions of murine cytotoxic T-lymphocytes ("CTL") in systems. Specifically, bryostatins 1 and 2 have been found to synergize with recombinant B-cell stimulatory factor ("BSF-1") to stimulate resting T-cells to proliferate and to differentiate into cytotoxic T-lymphocytes. Studies of in vivo primed spleen cells reveal that bryostatins dramatically decrease the concentration of interleukin-2 ("IL-2") that is needed for the development of CTL and enhance IL-2 induced development of cytotoxicity. Using cloned CTL both bryostatins 1 and 2, like 4 β-phorbol-12-myristate 13-acetate ("PMA") are shown to inhibit Ag-specific cytotoxicity but are able to bypass the requirement for TcR- crosslinking and can trigger exocytosis of cytolytic granules and cytotoxicity against non-Ag-bearing target cells. As used herein, TcR identifies the antigen receptor of T-lymphocytes. As will be shown, the present invention is predicated upon the use of bryostatin 1 and bryostatin 2, either alone or in combination with interleukin-2 plus B-cell stimulatory factor for immunomodulation in vivo.

Accordingly, a principle object of the present invention is to provide non-protein immunostimulators for enhancing the endogenous production of interleukin-2 and gamma-interferon within a patient's autoimmune system.

Another object of the present invention is to provide a non-protein immunostimulator which when administered intravenously to a host afflicted with a neoplastic growth simultaneously with cloned protein substances (interleukin-2 and/or gamma-interferon) substantially reduces the amount of such cloned foreign protein substance required to achieve a desired therapeutic effect thereby significantly reducing the risk of an anaphylactic reaction therefrom.

A further object of the present invention is to provide new and improved methods for activating protein kinase C, stimulating the endogeneous production of interleukin-2 and gamma-interferon within the autoimmune system of the host to whom it is administered.

Still another object of the present invention is to provide means for use in a new and useful method for administering cloned protein substances, such as interleukin-2 and/or gamma-interferon, in substantially reduced amounts to that heretofore required to achieve a given thereaupeutic effect, thereby minimizing the risk of an anaphylactic reaction therefrom, by the concurrent administration of a potent anti-tumor promotor protein kinase C activator therewith to potentiate the effect thereof.

A still further object of the present invention is to provide unique immunoenhancing substances for use in a method of treatment of neoplastic diseases whereby the autoimmune system of a host is activated to produce endogenous interleukin-2 and gamma-interferon thereby expanding the specificity of the T-cells (lymphocytes) in attacking neoplastic cells and preventing the spread of neoplasms without polluting the biochemical balance of the host by introducing foreign protein (e.g., cloned interleukin-2 and cloned gamma-interferon available, e.g., from Cetus Corporation, California) thereinto.

Still another object of the present invention is to provide unique immunoenhancing substances for use in a method pf treatment of neoplastic diseases whereby the autoimmune system of the host is augmented by the addition of a controlled amount of cloned interleukin-2 and gamma-interferon (foreign protein) thereto substantially concurrently with the injection of a small but effective amount of bryostatin 1 or bryostatin 2 whereby such controlled amount of such foreign protein is approximately 1/10 to about 1/20 of that required without the concurrent injection of bryostatin to obtain substantially the same therapeutic effect while reducing the risk of anaphylaxis and other known side effects of introducing foreign protein into the human system by about 10 to 20 fold.

These and still further objects as shall hereinafter appear are fulfilled by the present invention in a remarkably unexpected fashion as will be readily discerned from the following detailed description of exemplary embodiments thereof, especially when read in conjunction with the accompanying drawing.

In the drawings:
FIG. 1 shows the chemical structures of bryostatin 1 and 2;
FIG. 2 shows the effect of PK-C activators on the generation of cytotoxicity in primed spleen cells;
FIG. 3. shows the antigen specificity of bryostatin/IL-2 induced cytotoxic cells;
FIG. 4. shows the effect of bryostatin 2 on cytotoxicity of CTL clone against Ag-non-bearing target cells;
FIG. 5. shows the synergy of bryostatin with Ca⁺⁺ ionophores to induce specific BLT-E release from CTL clone;
FIG. 6. shows the inhibition of Ag-specific cytotoxicity of CTL clone by bryostatin 1 and 2; and
FIG. 7. shows the effect of bryostatin 1 and 2 and growth factors (IL-2, IL-4) on BLT-Esterase expression and cytotoxicity development in resting T-cells.

The present invention relates to newly discovered immunomodulating agents, herein denominated "bryostatin 1 and bryostatin 2", and methods of using them in the treatment of a host inflicted with neoplastic growth to either (a) stimulate the production of endogenous interleukin-2 and gamma-interferon within the autoimmune system of a host, which proteins being autogenerated are compatible with the biochemistry of the host; or (b) potentiate the activity of cloned interleukin-2 and gamma-interferon, both incompatible protein forms and therefore highly likely to elicit an antigenetic reaction which could result in anaphylaxis, thereby substantially reducing the amount of foreign protein required to obtain a desired therapeutic effect with the attendant benefit of significantly reducing the risk of untoward side effects from the use of a protein material which is not compatible with the biochemical makeup of the host.

As is known, cancer results from the inability of T-cells (in the host's autoimmune system) to recognize cancer cells and thereby permit them to expand unfettered within the host. It is believed that the use of the bryostatins in accordance herewith expands the range of T-cell recognition and thereby enables a result to be obtained by T-cell activity which otherwise would not occur. Further, the methods hereof achieve the result without employing high risk amounts of toxic materials such as foreign proteins and substantially minimize the risk of typical protein anaphylaxis, normally manifested by respiratory difficulties, sweating, fever and potentially, death.

Consider now the preparation of the bryostatins and the protocol demonstrating the totally unexpected benefits obtained therefrom in the following paragraphs.

### Isolation and Purification of Bryostatins 1 and 2.

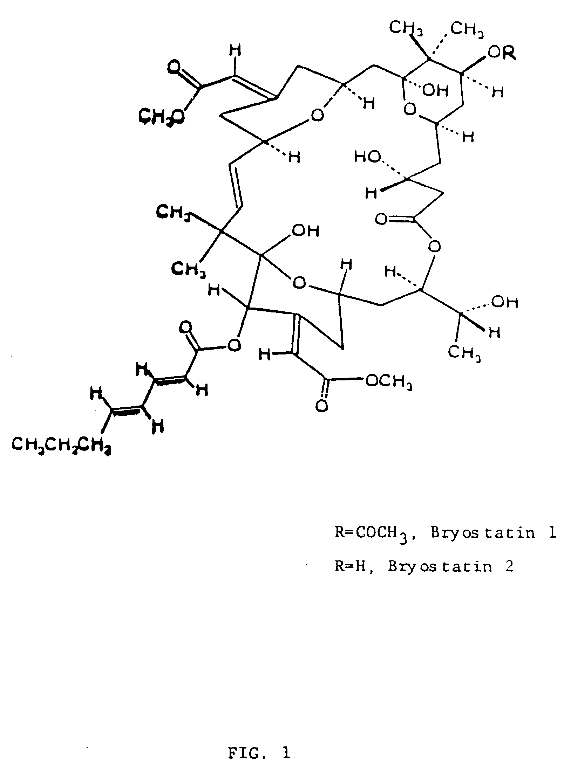

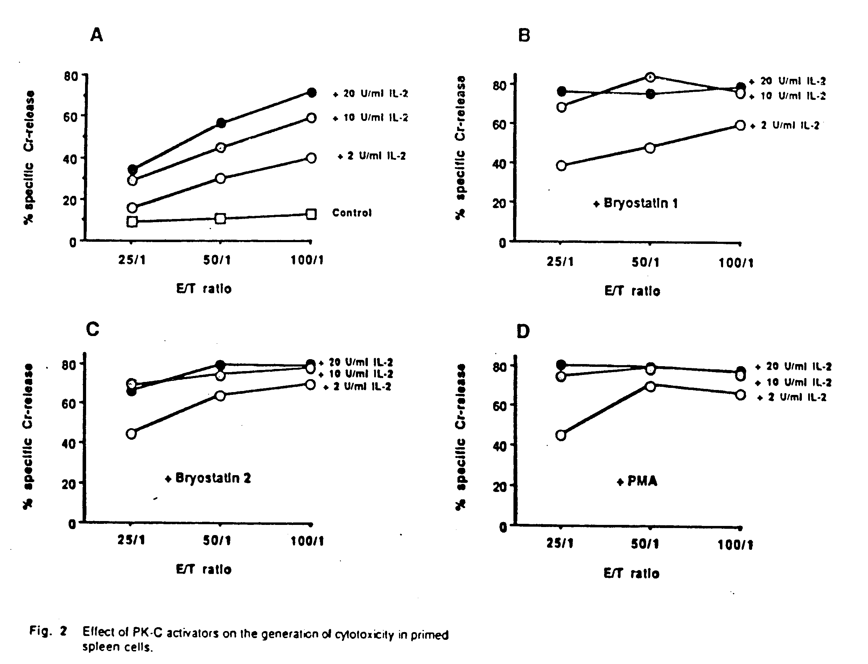

A methylene chloride extract prepared from 500kg (wet animals) Bugula neritina from the Eastern Pacific Ocean was further fractionated by the solvent partition sequence 9:1 4:1 methanol-water with ligroin-carbon tetrachloride followed by extensive column chromatographic techniques using both Sephadex LH-20 and silica gel as reported in Pettit, G. R., et al, supra, 1982, and Pettit, G. R., et al, 1983. The structure of bryostatin 2 from the marine Bryosoan Bugula neritina. J Natural Products 46:528. Both bryostatins are macrocyclic 20-membered ring lactones of very unusual structure as shown in FIGS. 1 and 2.

Bryostatin 2 is the 7-desacetyl-derivative of bryostatin 1.

### Animals.

Eight week old males DBA-2, B10 and BALB/c mice used throughout the study were purchased from Charles River, Frederick, MD.

### Cells.

The following cytolytic T-cell clones and target cells were used: cytotoxic T-lymphocyte ("CTL") OE4 (H-2^{b}, specific for H-2^{d}) (See: Staerz, U. D., et al, 1985. Hybrid antibodies can target sites for attack by T-cells. Nature 314:628), CTL 2C (H-2^{b}, specific for H-2^{d}) (See: Berrebi, G., supra); CTL BM10-37 (H-2^{d}, specific for H-2^{b})(See: Bluestone, J.A., 1987. Characterization of cytotoxic T-cell (CTL) clones derived from mutant H-2K^{bm10} anti-H-2K^{b} mixed lymphocyte culture populations: In Proceedings of the 15th International Leucocyte Culture Conference. Sussex, J. Willey & Sons, Ltd., pp. 149.); P815 (mouse mastocytoma cell line, H-2^{d}), EL-4 (T-cell lymphoma, H-2^{b}). Target tumor cells were maintained in RPMI 1640 medium supplemented with 10% FCS (Biofluids, Bethesda, MD). CTL clones were maintained as described in Glasebrook, A.L., and F.W. Fitch. 1980. Alloreactive cloned T-cell lines. I. Interactions between cloned amplifier and cytolytic T-cell lines. J. Exp. Med. 151:876, using irradiated stimulator cells and an IL-2- containing supernatant of concanavalin A-activated rat spleen cells.

### Cytotoxicity assay.

Effector cells and ⁵¹Cr-labeled target cells were mixed and incubated in triplicate in a 96-well plate at different E/T ratios as described in Grabstein, K. 1980. Cell-mediated cytolytic responses. In Selected Methods in Cellular Immunology, B. B. Mishel and S. M. Shiigi, eds. (San Francisco, Freeman), pp. 124. The target cell number was 10⁴ cells per well when ⁵¹Cr-release assay was performed with the cytolytic T-cell clones. 5x10³ target cells per well were used when spleen cells and lymph node T-cells were studied as effectors. PMA, bryostatin 1 and bryostatin 2 were added at a final concentration ranging from 0.5 ng/ml to 100 ng/ml. Lectin- dependent cytotoxicity assay was performed by adding 10 g/ml Con A to the mixture of CTL and ⁵¹Cr-labeled target cells. After incubation, the 96-well plate was centrifuged (1 min, 800 rpm, 4|C), all supernatants were harvested, radioactivity in the samples was counted and the mean values for each triplicate were determined. Percent of specific ⁵¹Cr release was calculated as 100X (a-b)/(t-b), where a is ⁵¹Cr release in the presence of CTL, b is spontaneous release from labeled target cells in the absence of CTL and t is the total number of cpm in the target cells. Spontaneous release of ⁵¹Cr from target cells did not exceed 15% of total cpm.

Estimation of the amount of cell-associated BLT-Esterase activity and BLT-Esterase secretion assay.

This assay was performed as described in Takayama, H., et al. 1987. Antigen receptor triggered secretion of a trypsin-type esterase from cytotoxic T-lymphocytes. J. Immunol. 138:566. Briefly, effector cells were resuspended in the incubation media and incubated in triplicate in a 96-well plate (Immulon) at a concentration of 10⁵ cells per 100 µl. Phorbol myristate acetate ("PMA"), bryostatin 1 and bryostatin 2 were added at a final concentration of 10 ng/ml, the Ca⁺⁺ ionophore A23187 (free acid, Sigma C7522) was added at a final concentration of 0.5 µ g/ml. Total volume per well was adjusted to 200 µl. After 4 hours of incubation (37°C, 5% CO₂) the plates were spun (1 min., 200 xG) and 50 µl of the supernatant was harvested. Total BLT-E content was determined by solubilizing 10⁵ effector cells with 0.1% Triton X-100 in PBS.

### Antibodies used for purification of small resting T-cells from murine lymph nodes or spleens.

The monoclonal antibody M5/114.15.2 (rat IgG_{2b}) directed against murine Ia antigens I-A^{b,d,q} and I-E^{d,k} (See: Bhattacharya, A., et al. 1981. A shared alloantigenic determinant on Ia-antigen encoded by the I-A and I-E subregions: evidence for I region gene duplication. J. Immunol. 127:2488.) was used at a final dilution of 1/100 ascites fluid together with a non-toxic rabbit complement (Cedar Line, Low- Tox-M rabbit complement, Ontario, Canada). The 7D4 rat IgM antibody to the mouse IL-2 receptor (See: Malek, T., et al. 1983. Identification and initial characterization of a rat monoclonal antibody reactive with the murine interleukin-2 receptor-ligand complex. Proc. Natl. Acad. Sci. USA 80:5694.) and MAR 18.5 (See: Lanier, L., et al. 1982. Monoclonal antibodies against rat immunoglobulin chains. Hybridoma 1:125.) mouse anti-rat kappa chain antibody have been previously described.

### Reagents:

rIL-2 was obtained from "Biogen" (Cambridge, MA). Recombinant B-cell stimulating factor (BSF-1IL-4) was provided by the Immunex Corporation.

### Purification of in vivo primed spleen cells.

B10 mice were immunized in vivo with 5-7.5x10⁶ irradiated P815 (H-2^{d}) cells. After 2-3 weeks animals were sacrificed and spleen cells were harvested. Red blood cells were lysed with hypotonic salt solution. Cells were incubated with 100 µl of 1/10 diluted M5/114.15.2 mAb containing ascites per 50x10⁶ cells. Following 30 min at room temperature, cells were washed and 100 µl of MAR 18.5 containing cell supernatant and 100 µl of rabbit complement per 50x10⁶ cells were added. Incubation at 37°C was allowed to proceed for 45 min., dead cells were removed by Hypaque-Ficoll centrifugation, washed and then incubated for three days in RPMI media/10% FCS with or without addition of IL-2, PMA or bryostatin as specified in legends of the drawing. Cytotoxicity against Ag-bearing target cells was measured in a ⁵¹Cr-release assay using P815 mastocytoma cells as targets.

### Purification of in vitro primed spleen cells.

Spleen cells were harvested from B10 mice and mixed with irradiated DBA-2 spleen cells at a 1:1 ratio. Cells were cultured in 5% FCS containing RPMI-1640 media at a concentration of 2x10⁶/ml. After 14 days in culture, cells were purified as described above.

### Purification of small resting T-cells from murine lymph nodes.

Mesenteric lymph nodes were harvested from virus-free BALB/c mice 8-12 weeks of age. After two nylon wool column passages, Ia positive cells were subsequently lysed with the anti-Ia monoclonal antibody M5/114 plus Low-Tox-M rabbit complement as described above. Small dense resting T-cells were then separated by a modified discontinuous Percoll gradient procedure (See: Defranco, et al. 1982. Frequency of B-lymphocytes responsive to antiimmunoglobulin. J. Exp. Med. 155:1523.). Purified T-cells which were harvested in the 66-70% density band were considered to be small resting T-cells. Staining with mAb followed by fluorescent sorter analysis revealed that virtually all of them were Thy 1⁺; 80% of cells were L3T4⁺ and 20% of cells were Lyt 2⁺.

### The effect of bryostatins 1 and 2 on the in vitro development of cytotoxic T-cells from spleens of in vivo-primed mice.

When purified T-lymphocytes from the spleens of in vivo primed mice were incubated in vitro in the absence of growth factors, maximum cytotoxic activity was detected at 24 hours; cytotoxicity decreased as cells were further maintained in culture (data not shown). Addition of increasing concentrations of IL-2 in the incubation medium resulted in an increasing number of lytic units in the population of murine spleen cytotoxic lymphocytes as shown in FIG. 2A and Table 1 below. Two U/ml of rIL-2/ml had only a small effect; maximum induction of cytotoxicity (51 lytic units per 10⁷ spleen T-cells) was achieved after 72 hours incubation of primed cells with 20 U/ml of rIL-2 as shown in FIG. 2A. However, when 0.78nM of bryostatin 2 or PMA were present during the incubation period, 2 U/ml of rIL-2 induced the development of CTL (see: Table 1, below) equal in magnitude to that induced by 20 U/ml of IL-2 as shown in FIG. 2. Furthermore, PMA and bryostatins 1 and 2 also enhanced the degree of cytotoxicity obtained with high concentrations of rIL-2.

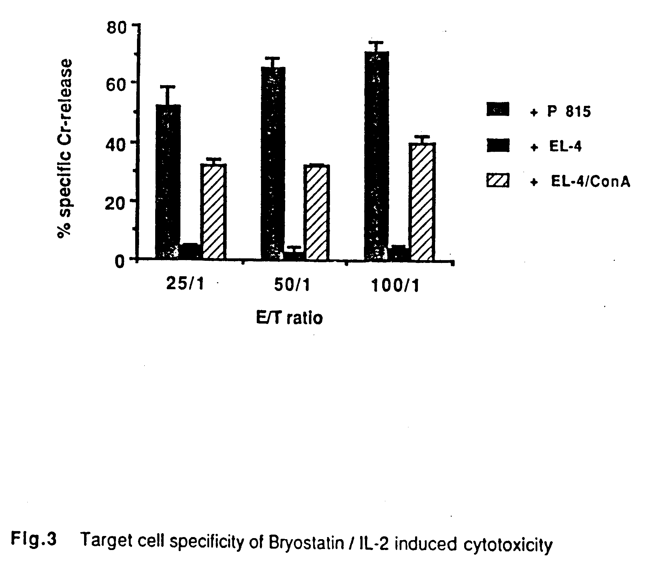

The cytolytic cells, which were stimulated by bryostatin and IL-2, were specific for the H-2^{d}-bearing target cells (see: FIG. 3), reflecting their development from mice primed with H-2^{d}-bearing cell line P815. No cytotoxicity against EL4 target cells was detected. However, bryostatin plus IL-2 induced cytotoxic cells were able to lyse EL-4 cells in the presence of Con A as shown in FIG. 3.

The enhancement of cytotoxic cell development by a bryostatin + IL-2 is strongly dependent on the concentration of bryostatin during the 3 day in vitro incubation of in vivo primed spleen cells. Bryostatin concentrations of 0.5 ng/ml or less enhanced IL-2 induced cytotoxicity; higher concentrations of bryostatin either had no effect (1 ng/ml) or inhibited IL-2-induced cytotoxicity (2 ng/ml or more)(data not shown).

### Triggering of Ag-nonspecific cytotoxicity of CTL clones in vitro by bryostatins 1 and 2.

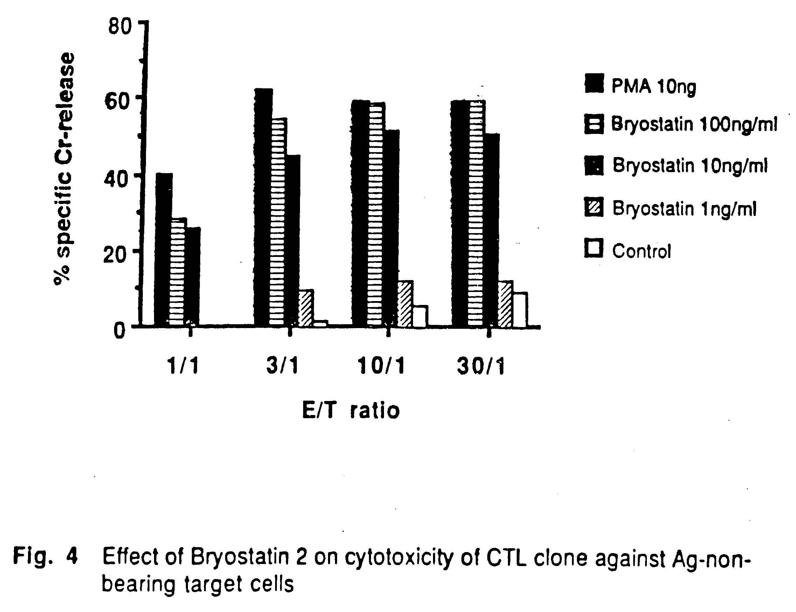

No ⁵¹Cr-release was observed when ⁵¹Cr-labeled EL-4 cells (H-2^{d}) were mixed with the cytotoxic T-cell clone 2C (anti-H-2L^{d}) at different E/T ratios. However, as shown in FIG. 4, the addition of bryostatins 1 or 2 to the cell mixture caused the lysis of EL-4 cells in a dose dependent manner, with maximal activity caused by 10 ng/ml of Bryostatin 2. It is believed that this represents a plateau of a dose-response dependence since a 10-fold increase in the concentration of bryostatin 2 did not result in a higher cytotoxicity of CTL. At 10 ng/ml of bryostatin 2 the level of ⁵¹Cr-release was consistently lower compared to the same concentration of PMA or bryostatin 1 at all E/T ratios tested (data not shown).

### Triggering of BLTE release from cytotoxic T-cell clone.

Since cytolytic activity of CTL is associated with the exocytosis of granules (See: Bykovskaja, S. N., et al. 1978. Ultrastructural alteration of cytolytic T-lymphocytes following their interaction with target cells. II. Morphogenesis of secretory granules and intracellular vacuoles. Cell. Immunol. 40:175; Zagury, D. 1982. Direct analysis of individual killer T-cells: Susceptibility of target cells to lysis and secretion of hydrolytic enzymes by CTL. Adv. Exp. Med. Biol. 146:149; Henkart, P. A. 1985. Mechanism of lymphocyte-mediated cyto-toxicity. Ann. Rev. Immunol 3:31; Russel, J. H. 1986. Phorbol-ester stimulated lysis of weak and nonspecific target cells by cytotoxic T-lymphocytes. J. Immunol. 136:23.; Takayama, H., et al, supra), the ability of bryostatins 1 and 2 to affect secretion of granule-associated trypsin-type esterase (BLT-E), in response to CTL interaction with specific target cell or following crosslinking of TcR by immobilized anti-TcR mAb was tested.

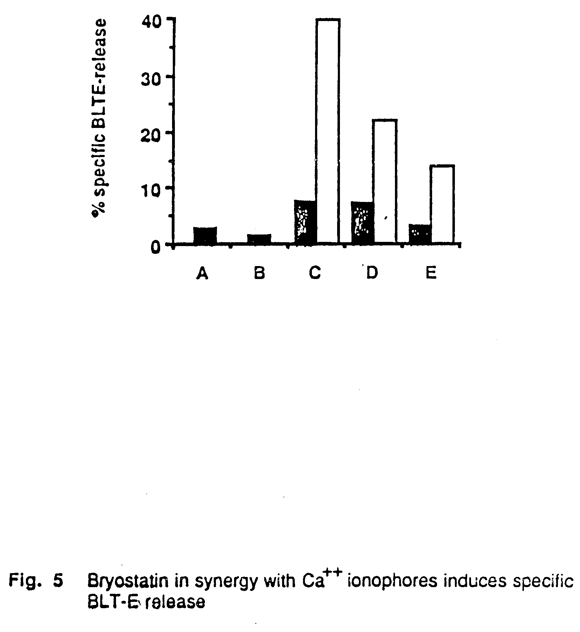

Using a newly developed BLT-esterase secretion assay (See: Takayama, H., et al, supra). the ability of bryostatins 1 and 2 to activate cytolytic T-cells and to trigger the exocytosis of granules from CTL was tested. As shown in FIG. 5, addition of bryostatin 1 or 2 alone induces only a slight increase in BLT-E secretion in the cytolytic T-cells. The combination of bryostatin and Ca⁺⁺-ionophore, however, greatly enhances the activation of CTL and induces significant secretion of granule located enzyme. PMA alone also induces a slight BLT-Esterase secretion which is greatly enhanced by 0.5 µg/ml of A23187. The effect of bryostatin 1/Ca²⁺ -ionophore is slightly lower than that of PMA/Ca²⁺-ionophore (as shown in FIG. 5). However, when equimolar concentrations of PMA and bryostatin are used, the amount of secreted BLT-Esterase was similar in both combinations. Bryostatin 2 or the combination of bryostatin 2/Ca²⁺ -ionophore was always less effective in the activation of cytotoxic T-cells than PMA and bryostatin 1, even at equimolar concentrations.

### Inhibition of specific cytotoxicity of CTL clones in vitro by bryostatins 1 and 2.

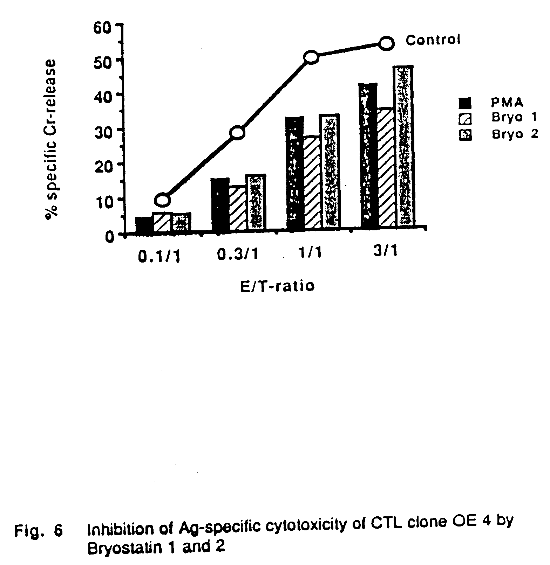

The effect of bryostatins 1 or 2 and PMA on Ag-specific cytotoxicity of CTL was tested in a ⁵¹Cr-release assay mixing P815 cells with CTL OE4 cells at various E/T ratios. As shown in FIG. 6, specific ⁵¹Cr-release was decreased when 10 ng/ml of bryostatin 2 was added to the samples. Similar inhibition was achieved by the addition 10 ng/ml of PMA. The same concentration of bryostatin 1, however, resulted in a slightly but consistently higher inhibition of Ag-specific ⁵¹Cr-release compared to PMA. Pretreatment of CTL with PMA and bryostatin instead of adding them directly into incubation wells of a microtiter plate did not significantly change described effects.

### Effect of bryostatins 1 and 2 on the development of cytolytic granules and of cytotoxicity in small resting T-lymphocytes.

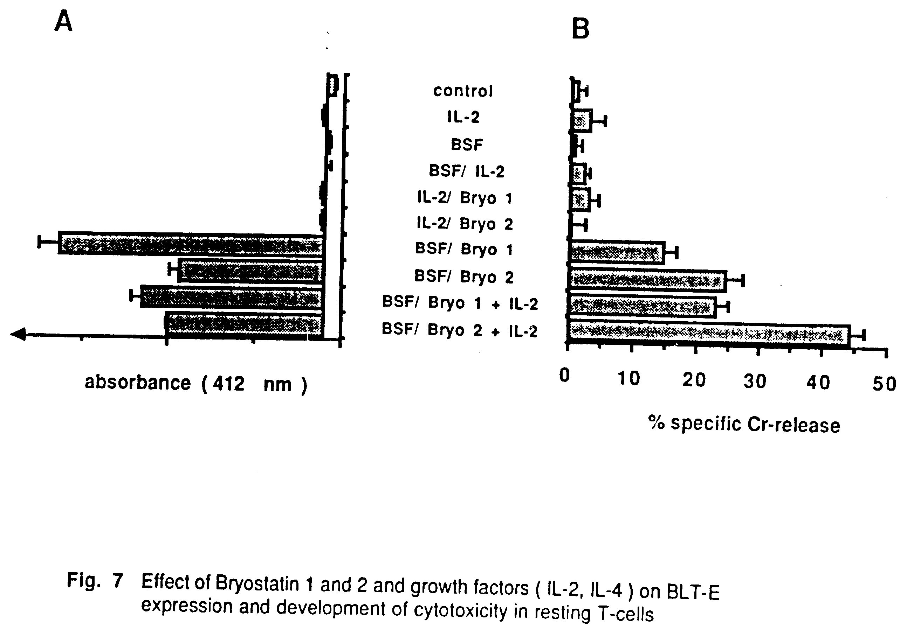

Strong enhancing properties of bryostatin were found when effects of PMA, bryostatin 1 and bryostatin 2 on small resting unprimed cytotoxic T-cells were measured. Previous studies by Trenn, G., et al demonstrated that small resting cytotoxic T-cells are effectively differentiated in mature cytotoxic T-cells by the simultaneous addition of BSF-1 and PMA to the culture medium, which effect is enhanced by the addition of IL-2. The accumulation of CTL granule marker, BLT-E (See: Pasternack, M. S., et al. 1985. A novel serine esterase expressed by cytotoxic T-lymphocytes. Nature (Lond) 314:743.) during incubation of naive resting T-cells with growth factors and protein kinase C activators were measured and the results are shown in FIG. 7, panel A. Neither growth factors (IL-2, IL-4) nor bryostatin 1 and 2 alone induced expression of BLT-E activity in resting T-cells. However, bryostatins 1 and 2 in combination with IL-4, but not IL-2, dramatically increased the amount of this granule associated enzyme on a per cell basis, reflecting differentiation of resting T-cells into CTL. Ability of bryostatin to enhance the development of CTL is supported by the studies of the effect of bryostatins 1 and 2 on development of cytotoxicity in resting T-cells as revealed in a ⁵¹Cr-release assay reported in FIG. 7, panel B. Bryostatins 1 and 2 alone as well as BSF-1 and IL-4 alone failed to induce CTL development, while they synergized when added together. Interestingly, in ⁵¹Cr-release assay (FIG. 7, panel B) addition of IL-2 enhanced the effect of BSF-1/bryostatin, while this was not the case when BLT-E amount per cell was measured after incubation of cells in the same conditions (FIG. 7, panel A).

From the foregoing, it can be discerned that potent immunoenhancing properties of a novel series of protein kinase C activators selected from the bryostatin family are demonstrated. In contrast to the PK-C activating phorbol esters, which have potent tumor promoting activity, the bryostatins lack tumor promoting activity and have demonstrated anti-neoplastic properties (See: Pettit, G.R., et al., 1982, supra; Pettit, G. R., et al, 1983, supra; and Pettit, G. R., et al, 1984, supra). Important cell-biological differences between PMA and bryostatin have been observed, including differences in their effects on immature cell lines. It has been shown that differentiation of the HL-60 cell line can be induced by PMA but not by the bryostatins (See: Kraft, A.S., et al. 1986. Bryostatin, an activator of the calcium phospholipid-dependent protein kinase, blocks phorbol ester-induced differentiation of human promyelocytic leukemia cells HL-60. Proc. Natl. Acad. Sci. 83:1334.). Thus, although activation of protein kinase C is sufficient to induce many phorbol ester-like effects, modulation of differentiation may be more complex. It appears that bryostatin, a macrocyclic lactone, elicits different effects on intracellular biochemical pathways than those elicited by PMA, and that the duration of its effects may differ. Differences in the protein phosphorylation pattern induced by PMA and the bryostatins have been reported (See: Berkow, R. L., et al. 1985. Bryostatin, a non-phorbol macrocyclic lactone, activates intact human polymorphonuclear leukocytes and binds to the phorbol ester receptor. Biochem. Biophys. Res. Commun. 131:1109).

As described above and shown in FIGS. 2-7, bryostatins 1 and 2 resemble PMA in that they are potent modulators of the response of both resting and in vivo primed T-cells and of cloned T-lymphocytes. Further, these bryostatins enhance the development of cytotoxic cells from naive resting T-cells (see: FIG. 7), and from in vivo primed spleen cells (see: FIG. 2, and Table 1). The bryostatins also promote Con A-dependent cytotoxicity against target cells lacking determinants for which the CTL are specific as shown in FIG. 4. Quantitative studies of the exocytosis of cytolysin-containing granules from CTL show (see: FIG. 5) that either the phorbol ester or the bryostatins can synergistically enhance exocytosis of granules from CTL stimulated with Ca⁺⁺ ionophores.

Bryostatins synergize with lymphokines in the stimulation of naive T-lymphocytes as shown using purified small resting T-cells from murine mesenteric lymph nodes. Bryostatin and IL-4 cause CTL-precursors in this resting T-cell population to differentiate into cytotoxic T-cells (FIG. 7). This process of maturation is enhanced by the addition of IL-2 and is accompanied by the development of the granule associated trypsin-like serine esterase BLT-E (FIG. 7A). Although in some experiments bryostatin 1 is less effective than PMA in induction of cytotoxicity in small resting T-cells, bryostatin 2 consistently induced similar amounts of cytotoxicity as shown in FIG. 7B.
The studies of the effect of bryostatins on in vivo primed cytotoxic T-cells, as shown in FIGS. 2 and 3 and Table 1, demonstrate that low doses of IL-2 (2 Units/ml) plus bryostatin can induce as many, or more, CTL lytic units as will high doses of rIL-2 (10 or 20 units per ml). This effect is not due to the additional release of endogenous IL-2 from stimulated helper T-cells, since IL-2 induced proliferation in the cell culture was not detected when cells were incubated with IL-2 plus bryostatin. Thus, bryostatin significantly decreases the amount of rIL-2 that is needed for the development of CTL thereby reducing the risk of untoward side effects. Thus, it is believed that the use of bryostatin with rIL-2 in accordance herewith will allow tumor rejection (See: Rosenberg, S. A., et al. 1987. A progress report on the treatment of 157 patients with advanced cancer using lymphokine-activated killer cells and interleukin-2 or high-dose interleukin-2 alone. New Engl. J. Med. 316:889) with IL-2 doses sufficiently low to mitigate or avoid the undesirable side effects (See: Moertel, C. G. 1986. On lymphokines, cytokines, and breakthroughs. JAMA 256:3141) of high doses of rIL-2 currently used in adoptive immunotherapy. Bryostatin thus is attractive as an immunomodulating agent because it not only displays antineoplastic properties, but also has anti tumor-promoter activity.

Detailed studies of the effect of bryostatins on lymphocyte growth, differentiation and effector functions indicate that they are not lymphotoxic. It was consistently found that the percentage of dead cells in bryostatin containing cultures of CTL after even three days of incubation did not differ from the control culture (estimated by trypan blue staining). No lymphotoxic effect of bryostatin has been found using the LDH secretion assay or trypan blue exclusion assay in CTL cultures containing even higher concentrations of bryostatin. However, while bryostatins enhanced the IL-2 induced development of murine CTL-cells after 24 or 72 hours in culture (data not shown), they did not enhance overall cell recovery after 72 hours in vitro incubation of in vivo primed spleen cells and inhibited IL-2 induced increases in index of cell recovery as shown in Table 1, below.

One of the interesting properties of protein kinase C activators including bryostatins, as shown in FIG. 6, is their ability to inhibit Ag-specific cytotoxicity of CTL clones. Thus it is demonstrated that bryostatins 1 and 2 are PK-C activators with advantageous properties for use as immunomodulatory agents.

**Table 1**

| Effect of bryostatin on the generation of cytotoxicity in primed spleen cells | | |
|---|---|---|
| Culture Conditions | Lytic Units Per 10⁷ cells^{a} | Index of Cell Recovery after incubation^{b} |
| Media | <7 | 1 |
| Media + rIL-2 (2U/ml) | 10 | 2 |
| Media + rIL-2 (10U/ml) | 33 | 2.2 |
| Media + rIl-2 (2U/ml) + PMA^{c} | 71 | 1.3 |
| Media + rIL-2 (2U/ml) + Bry 1 | 40 | 0.9 |
| Media + rIL-2 (2U/ml) + Bry 2 | 70 | 1.0 |
| Media + rIL-2 (20 U/ml) | 51 | 2.2 |

| | | |
|---|---|---|
| ^{a}Lytic unit is defined as the number of effector cells necessary for 50% specific ⁵¹Cr-release from 10⁴ target cells. | | |
| ^{b}Index of cell recovery was calculated as ratio of cell number in the beginning of the culture to the cell number after 3 days of incubation. Primed spleen cells were obtained as described in Example I. | | |
| ^{c}0.5 ng/ml PMA or 0.5 ng/ml bryostatin 1 were used during incubation of cells when indicated. | | |

To further aid in the understanding of the present invention and not by way of limitation, the following examples are presented.

### EXAMPLE I

In vivo primed B10 spleen cells were prepared as described above. After a 3-day culture either in the presence of either rIL-2 or rIL-2 plus protein kinase C activator, cytotoxicity was measured in a ⁵¹Cr release assay at the E/T ratios shown in FIG. 2 using 10⁴ P815 target cells/well. Results of cytotoxicity assay are shown for (A) cells cultured with rIL-2 alone (0 U/ml, 2 U/ml, 10 U/ml, 20 U/ml); (B) cells cultured with rIL-2 plus 0.5 ng/ml bryostatin 1; (C) cells cultured with rIL-2 plus 0.5 ng/ml bryostatin 2; and (D) cells cultured with rIL-2 plus 0.5 ng/ml PMA. All results are displayed in FIG 2.

### EXAMPLE II

B10 mice Were immunized with P815 cells as described above. Ia-cells depleted spleen cells were incubated in the presence of 0.5 ng/ml bryostatin 2 and 10 U/ml rIL-2. After three days, cytotoxicity was checked in a ⁵¹Cr release assay, using 10⁴ P815 (H-2^{d}) or EL-4 (H-2^{b}) target cells. Con A was added to a final concentration of 10 µg/ml. The results obtained are displayed in FIG. 3.

### EXAMPLE III

CTL 2C clone were incubated 4 hours with ⁵¹Cr-labeled syngeneic EL4 target cells at different E/T ratios in the presence of different concentrations of PMA or bryostatin 2. The results are shown in FIG. 4.

### EXAMPLE IV

10⁵ CTL (clone BM10-37) was incubated in a 96-well plate and stimulated by the addition of a PMA (10ng/ml) and A23187 (0.5 µg/ml). Specific BLT-E secretion was measured as described above. The results are displayed in FIG. 5 using the following code: A is media plus solvent DMSO; B is A23187 (0.5 µg/ml) alone; C is PMA (10 ng/ml) plus A23187 (0.5 µg/ml); D is bryostatin 1 (10 ng/ml) plus A23187 (0.5 µg/ml); E is bryostatin 2 (10 ng/ml) plus A23187 (0.5 µg/ml).

### EXAMPLE V

OE4 CXTL cells were incubated with 10⁴ Cr labeled P815-cells at different E/T ratios and a ⁵¹Cr-release was measured after 4 hours incubation. PMA or bryostatin was added to a final concentration of 10 ng/ml. The results are shown in FIG. 6.

### EXAMPLE VI

Small resting T-cells were purified from Balb/c mesenteric lymph nodes as described above. Purified cells were cultured for 3 days at a cell concentration of 10⁶ cells/ml either in media alone or in media plus different additions (rIL-2,100 U/ml; BSF-1, 2000 U/ml; bryostatin 1 or 2, 0.5 ng/ml). Cytotoxicity was measured in a 6 hour lectin mediated (Con A, 10 µg/ml) ⁵¹Cr-release assay. Results are shown for an E/T ratio of 100/l. For the determination of BLT-E content, 10⁶ cells of each of tested culture conditions were resuspended in 100 µl of media and solubilized with 1% Triton® X-100. Fifty l of cell suspension was used for measurement of BLT-esterase content as described above. Tested reagents when added alone to the cell cultures neither increased cellular BLT-esterase content nor induced cytotoxicity as shown in FIG. 7.

## Claims

1. A composition comprising (i) a compound of structure: 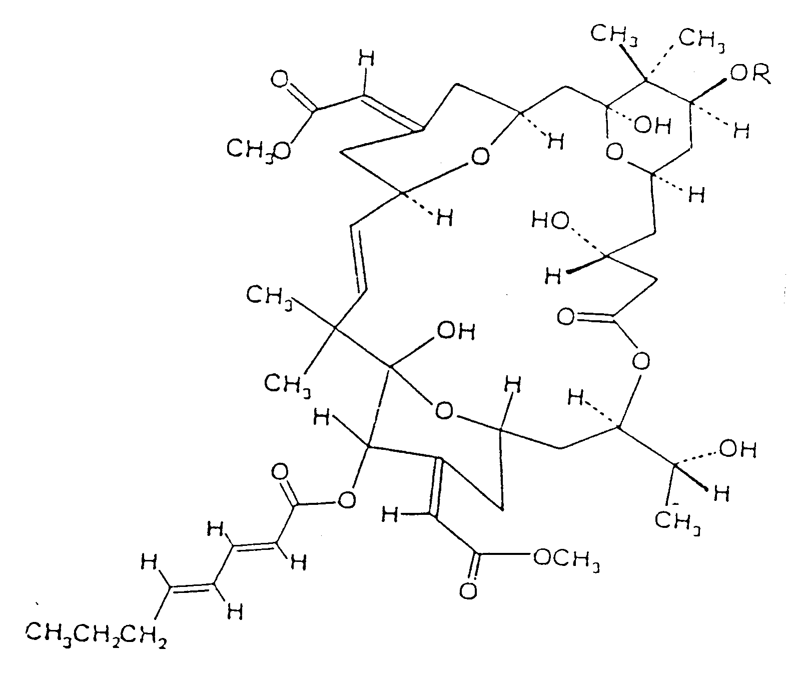 wherein R is -H or -COCH₃
and (ii) cloned interleukin-2 or gamma-interferon.

2. The use of a compound of structure: 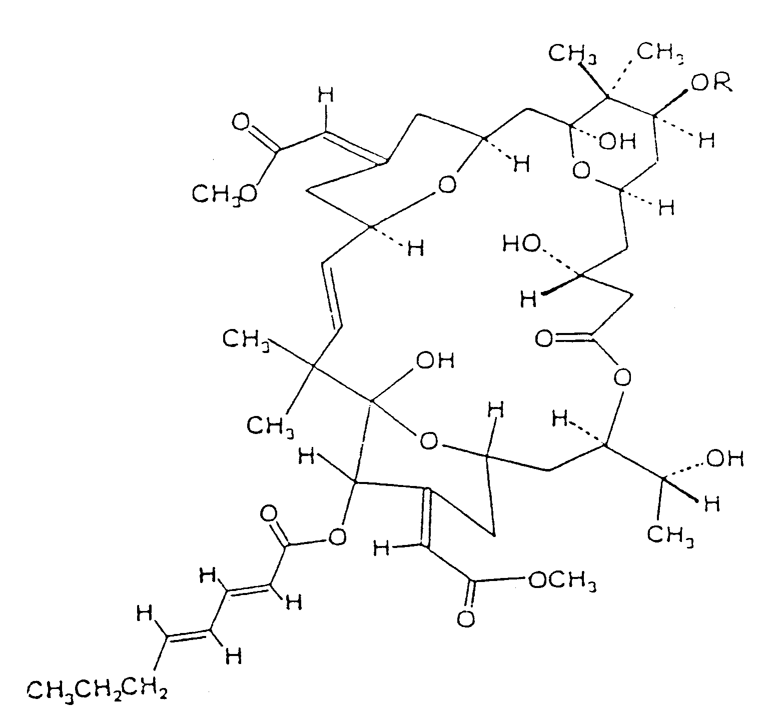 wherein R is -H or -COCH₃,
in the preparation of a medicament for treating a host afflicted with neoplastic growth, the host being treated also with cloned protein interleukin-2 and/or gamma-interferon.

3. A use according to Claim 2 wherein said medicament is suitable for administration by intravenous injection.

4. A use according to Claim 2 or 3 wherein R is H.

5. A use according to Claim 2 or 3 wherein R is COCH₃.

## Patentansprüche

1. Zusammensetzung, enthaltend (i) eine Verbindung der Struktur: 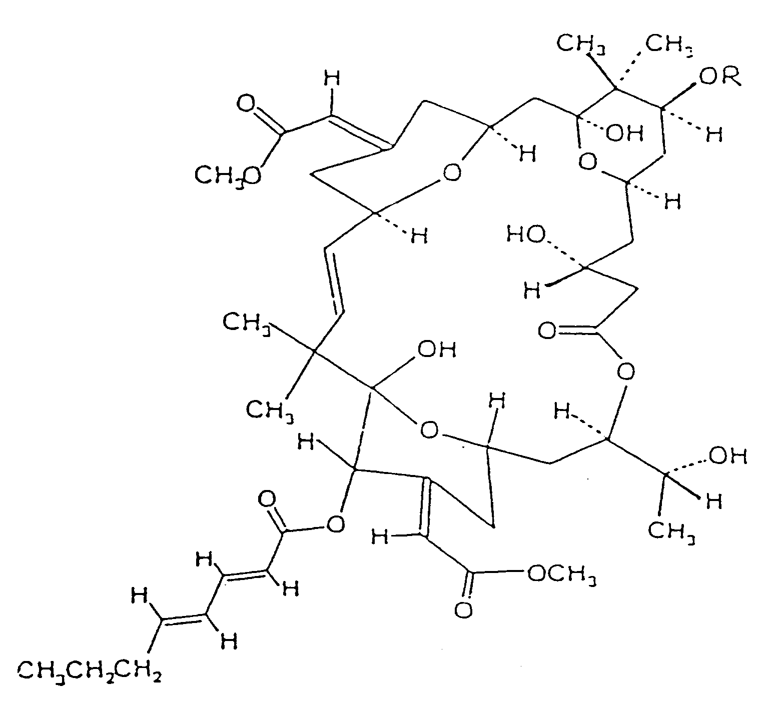 worin R -H oder -COCH₃ ist,
und (ii) kloniertes Interleukin-2 oder gamma-Interferon.

2. Verwendung einer Verbindung der Struktur: 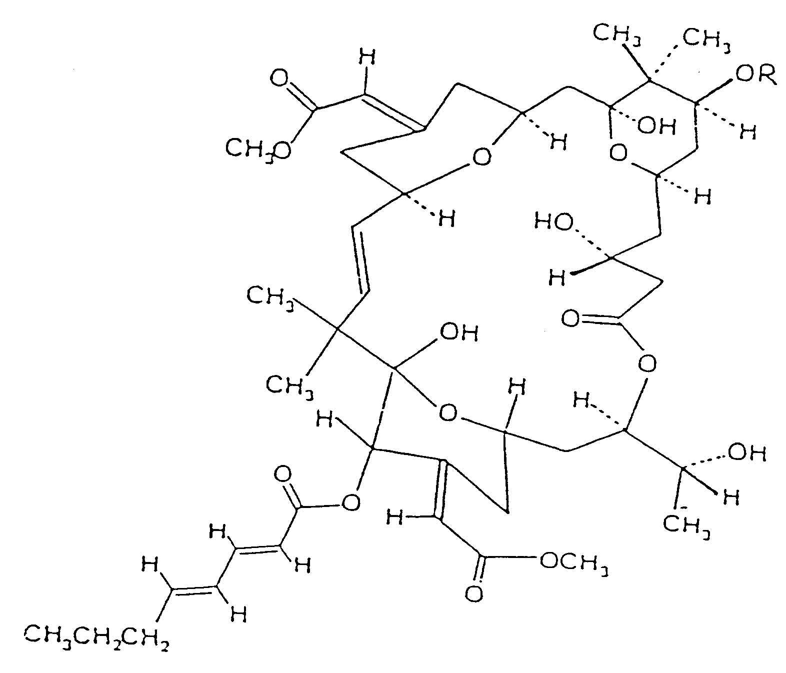 worin R -H oder -COCH₃ ist,
bei der Herstellung eines Medikamentes zum Behandeln eines Wirtes, der unter neoplastischem Wachstum leidet, wobei der Wirt außerdem mit kloniertem Protein Interleukin-2 und/oder gamma-Interferon behandelt wird.

3. Verwendung nach Anspruch 2, worin das Medikament für die Verabreichung durch intravenöse Injektion geeignet ist.

4. Verwendung nach Anspruch 2 oder 3, worin R H ist.

5. Verwendung nach Anspruch 2 oder 3, worin R COCH₃ ist.

## Revendications

1. Composition comprenant (1) un composé répondant à la formule : 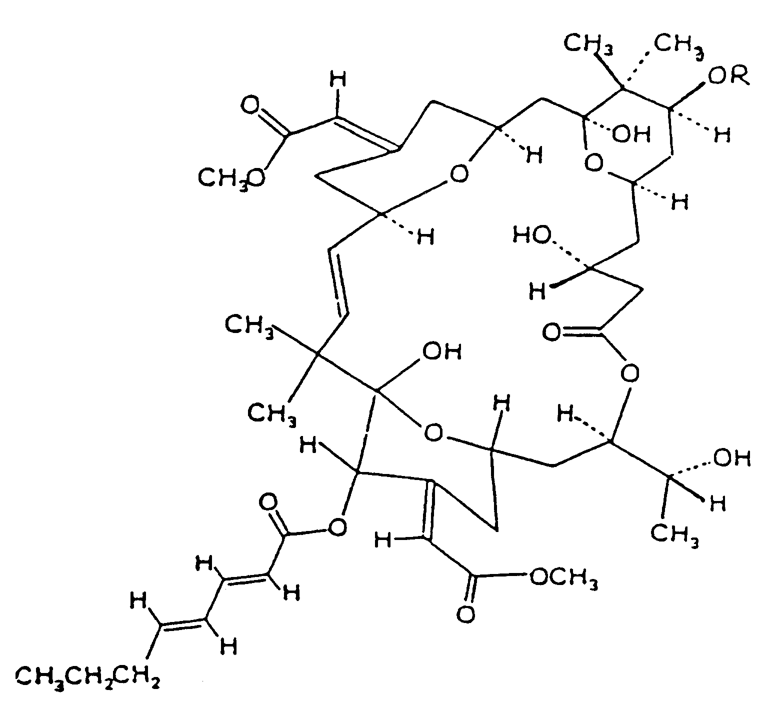 dans laquelle R est -H ou -COCH₃,
et (2) de l'interleukine-2 ou de l'interféron γ clonés.

2. Utilisation d'un composé répondant à la formule : 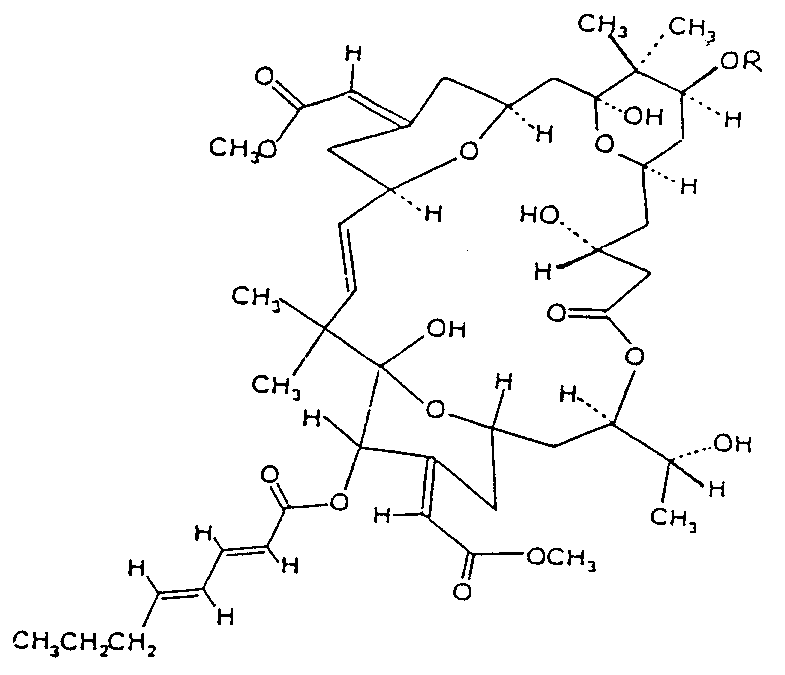 dans laquelle R est -H ou -COCH₃,
pour la préparation d'un médicament pour le traitement d'un hôte souffrant d'une croissance néoplasique, l'hôte étant traité par les protéines clonées interleukine-2 et/ou interféron γ.

3. Utilisation selon la revendication 2, dans laquelle ce médicament convient pour l'administration par injection intraveineuse.

4. Utilisation selon la revendication 2 ou 3, dans laquelle R est -H.

5. Utilisation selon la revendication 2 ou 3, dans laquelle R est -COCH₃.
